# EUROPEAN PATENT APPLICATION

(11) **EP 2 103 686 A1**
(43) Date of publication of application: **23.09.2009**
(21) Application number: 08425170.1
(22) Date of filing: 17.03.2008
(51) Int. Cl.: C12N 5/06

(54) **Biotechnological process for isolation of hepatocytes from marine organisms**

(71) Applicant: Universita' Degli Studi di Bari, 70121 Bari (IT)
(72) Inventor: Centoducati, Gerardo, 70122 Bari (IT); Santacroce, Maria Pia, 70123 Bari (IT); Conversano, Maria Chiara, 73100 Lecce (IT); Crescenzo, Giuseppe, 84098 Pontecagnano (SA) (IT)
(74) Representative: Bruni, Giovanni

(57) **Abstract**

biotechnological process for preparing primary hepatocyte cultures from the liver of marine water species characterised by a step of preparing the liver followed by a triturating/filtration step, then an enzymatic digestion, purification through differential and isopycnic centrifugation and lastly a cellular resuspension in complete medium.

## Description

The objective of the present finding is a biotechnological process, a method for preparing primary hepatocyte cultures from the liver of marine water species in a special implementation such as primary hepatocyte culture of European sea bass (*Dicentrarchus labrax* L.) and gilt-head bream (*Sparus aurata* L.) liver. The development of primary cultures and/or cell lines from water organisms represents a key instrument for applications in marine biotechnologies, in epidemiological, molecular carcinogenesis, toxicological and functional genomic studies. Various cell lines have been developed from freshwater toleostei, while the number of those isolated from marine species is relatively low. In particular, over the last decade the scientific community has focused more and more attention on the fish species most reared and sold in Europe, such as *D. labrax* and S. *aurata*. Among the experimental animal models based on *in vitro* systems, primary hepatocyte cultures are considered as the *gold standard* for studies regarding the biosynthetic and metabolic cell functions. According to literature, currently available are processes for preparing primary hepatocyte cultures basically produced by the freshwater species, the most commonly studies being, first and foremost the Rainbow trout (*Oncorhynchus mykiss*). Regarding the water vertebrates, the trout represented the first model of study for the preparation of cell cultures: the first isolations and culture protocols, dating back to the early 70s, resulted from a reproduction of rat liver hepatocyte isolation, by means of the *in situ* two-stage collagenase hepatic perfusion technique. According to the known art, the only hepatocyte isolation from *Sparus aurata* known up to date, indicated in literature, refers to the perfusion method as described by Mommsen and Bevelander (Mommsen T.P., Moon T.W. and Walsh P.J., 1994, Hepatocytes: isolation, maintenance and utilization, Biochemistry and molecular biology of fishes 3: 355-373; Bevelander G.S., Hang X, Abbink W, Spanings T, Canario A.V.M., Flik G., 2006, PTHrP potentiating estradiol-induced vitellogenesis in sea bream, Sparus auratus, L., General and Comparative Endocrinology 149: 159-165). The hepatic perfusion process is the most common method, not only for mammal models, but also for some water vertebrate species. However, this process has some drawbacks: poor availability of hepatic cell lines deriving from marine teleostei, the method is long and overly analytical during the animal preparation step and requires the use of specific equipment (a pump with a calibrated injection system to keep the pressure and the enzymatic fluid flow constant, as well as a washing system, in the time unit). Furthermore, the hepatic perfusion process requires operating with the animal still alive, sedated and dissected, with an open peritoneal cavity. Though wishing to avoid raising issues regarding ethical values, the long waiting periods for vein washing, manoeuvres for inserting cannulas and for clamping operations, expose such method to potential risks of airborne, intestinal or bile contaminations jeopardising the isolation quality. The objective of the present finding attains the preset objectives in that it is a biotechnological process adapted to produce a system, per se not found in nature, in a laboratory, based on the extraction of living units of the animal in question and on their assembly in a new system useable for scientific research. In this case, human intervention consists in transferring an actual living being, from the natural environment to the laboratory, maintaining the same characteristics and biological processes which shall be subjected to interventions to study their various response effects.

The finding subject of the present invention attains the preset objectives in that it is a method for preparing primary hepatocyte cultures from the liver of marine water species comprising the following steps:
- Preparing and washing the liver;
- Triturating/Filtering the explanted hepatic tissue using sets of steel filters;
- Enzymatic digestion using a mixture of enzymes, suitably selected according to their distinctive selectivity and activity;
- Purification of the hepatic cells, through differential and isopycnic centrifugation on density gradient;
- Resuspension of purified cells, cell count, viability control, plating and incubation in a complete medium;
- Maintaining the hepatic cells in culture, through optimisation of the growth conditions, choice of the adhesion substrate, the culture medium and the physical/chemical parameters suitable to allow the growth of the marine cells.

More in particular, the finding in question consists in a method allowing a more rapid and less analytical process for isolating, purifying, and maintaining selectively in culture - over a long period of time - the hepatocytes derived from the explanted liver of marine toleostei belonging to the *D. labrax* and *S. aurata* species, through different steps of physical and enzymatic disassociation as well as cell purification.

These and other advantages shall be observed from the detailed description of the invention with specific reference to table 4 bearing a summary flow chart of the process in question, strictly provided for exemplification and non-limiting purposes.

With reference to the abovementioned table, shown is a summary diagram of the experimental protocol of the preparation of primary liver hepatocyte cultures from marine water species, such as the gilthead sea bream and the European sea bass, in a special implementation such as hepatocyte from the gilthead sea bream liver culture.

Said process comprises the following steps:
1) Isolation step comprising the stages of : preparing and washing the liver, mincing/filtering and enzymatic digestion;
2) Purification step comprising the stages of: differential and isopycnic centrifugation:
3) Plating step comprising the stages of: cell count, viability control, resuspension of the hepatic cells in complete medium, seeding in microplates pre-treated with collagens matrix, incubation, in a CO2 refrigerated incubator, adhesion to the matrix;
4) Maintenance step comprising the addition of the differentiation medium maintaining the differentiated phenotype in culture over a long period of time.

The process starts by preparing the liver: young specimen of gilthead sea bream are sacrificed in an ice bath and immediately weighed and decerebrated, by incising using a sterile long bladed scalpel, at a supraocular position. The bodies are arranged in a sterile petri dish with the cranial part facing to the left in such a manner to perform all the dissection operations which allow exposing the liver open. All the dissection operations are performed fastest possible in such a manner to limit the occurrence of any ischemic conditions capable of damaging the hepatic parenchyma. After spraying 70% ethanol on the operative side (incision surface), a longitudinal incision is performed, in an anteroposterior direction (sterile straight bladed disposable scalpel) in the lower abdomen, followed by a dome incision which, following the back side line, intersects the abdomen incision at the anal level. This allows overturning the skin surface and the separation of the muscles. At this point, the intestinal mass, the kidney, and the gonads are removed from the abdomen to clear the dissection area (sterile curve bladed disposable scalpel). The isolated liver is thus placed on a sterile steel spoon, cautiously freed of any hepatic ligaments and of gallbladder and then weighed in a sterile petri dish. Once transferred to a second petri dish containing washing buffered saline, next follows the extraction of the Glisson membrane and the removal of the large vessels (thin #55 micro-dissection forceps). The liver, suitably dissected in step 1, is subjected to external washing, by submersion in a buffered saline free of calcium and magnesium ions (5ml/liver) and internal washing, by means of a syringe until complete blood clarification is obtained (Washing Buffer n° 1, in Table 1). The removal of bivalent cations allows both freeing of the intercellular bonds, hence facilitating the disassociation of the parenchyma and preventing the deterioration of the hepatocytes under the action of the protease possibly released by the damaged tissues.

**Table 1**

| **Washing buffer n° 1 in HBSS** | | |
|---|---|---|
| (5 ml/liver, Mg⁺⁺ and Ca⁺⁺ free) | | |
| **Washing Medium:** | **Final concentration** | **Initial concentration** |
| EDTA | 0.5mM | 0.5M |
| Pen/Strep 2X | 200 µg/ml | 10000 µg/ml (100x) |
| Gentamicin 2X | 0.05 mg/ml | 10 mg/ml |
| Hepes | 10 mM | 1M |
| Na₂HCO₃ | 25mM | 0.89M |
| HBSS (Hanks' balanced salt solution) | | |

Next follows the tissue disintegration step by using a #40 (380 µm, S 0770-Sigma) stainless steel filter, mounted on a sterile steel colander (S 1145-Sigma), and a glass pestle (T 8279-Sigma) for softly pressing the hepatic masses through a rotational movement. In order to allow the passage of the homogenised tissue small doses of digestion buffer (Buffer n° 2 in Table 2), are regularly added reaching 10 ml/g of final liver volume.

**Table 2**

| **Digestion buffer in Liebovitz L-15, n.2** | | |
|---|---|---|
| **Digestion buffer:** | **Final concentration** | **Initial concentration** |
| CaCl₂ | 7mM | 2 M |
| Pen/Strep/Amph 2X | 200 µg/ml | 10000 µg/ml (100x) |
| Gentamycin 2X | 0.05 mg/ml | 10 mg/ml |
| Hepes | 10 mM | 1M |
| NaHCO₃ | 12mM | 0.89 M |
| Collagenase | 0.1% | 1% |
| Hyaluronidase | 0.05% | 2% |
| Dispase | 0.4% | 2.5% |
| DNase I | 0.03% | 1% |

Once through with the mincing/filtration step, next follows the enzymatic digestion. A mix of enzymes is added to the homogenate at different enzymatic activities and final concentration: Collagenase 0.1% (Type IV, Activity 400-600 U/mg; C-5138, Sigma)/Hyaluronidase 0.05% (Type IV-S, 750-1500 U/mg; H3884, Sigma)/Dispase 0.4% (Type II, D4693, Sigma)/DNase 0.03% (Type I, 400-800 Kunitz U/mg; DN25, Sigma), then it is left to incubate for 20 minutes at 18-20°C, under a low stirring (10 rpm) on an orbital shaker (Barloworld Scientific, Milan). At the end of the enzymatic digestion stage, 20 mls of Liebovitz L-15/FBS 10% (inactivated e decomplemented a 56°C) are added, to block the residual enzymatic activity. Following are two further filtration stages through #60 (230 µm; S 1020 Sigma) and #150 (104 µm; S 4020 Sigma) steel filters. Next follows the purification step 2 through differential and isopycnic centrifugation. The cells thus disassociated are subjected to washing (cell suspension/PBS at a 1:3 ratio) in order to remove the connective microfilaments, the fragments or broken cells and the erythrocytes, through 4 sequential centrifugations at 4°. The first centrifugation is performed at 80 g (900 rpm) for 5' in a refrigerated centrifuge (Sanyo Harrier 18/80), with a fixed tilted arm rotor.

Then, follows the recovery of the pellet obtained from centrifugation I through a fine tipped glass pasteur, suctioning the whiter portion of the pellet free of erythrocytes in a very slow manner. The recovered pellet is transferred to another centrifuge tube (50 ml sterile falcon) and washed, pipetting several times and very cautiously, with cold PBS. For better separation of the parenchyma cells from the non-parenchyma ones and from the erythrocytes, an isopycnic separation by means of density gradient centrifugation gradient (centrifugation II) is performed. The cell suspension, added with Liebovitz L-15 (1:1, v/v), is stratified very cautiously (drop by drop) on a double density gradient (1:2, v/v, cell susp./percoll) of Percoll at 90%-50% (2:1, v/v) in PBS (10X). The 50 ml falcons with double gradient are prepared before the isolation and maintained at 4°C up to the use. The centrifugation on gradient is performed at 150 g (1400 rpm) for 10 minutes at 4°C. At the end a clear stratification of cell bands can be observed: the surface layer of the buffy coat (layer I ) contains a surface portion of dead and fragmented cells, followed beneath a white ring of isolated cells (layer II ) which lies on a red ring of lysed erythrocytes (layer III) positioned in close contact with the Percoll. After having cautiously removed the first layer directly, by suctioning with a fine-tipped pasteur the white hepatocyte ring is carefully recovered avoiding suctioning the erythrocytes ring. The new recovered cell suspension is further washed in cold PBS (1X) through two further centrifugation stages at 70 g (788 rpm) for 5' at 4°C. Lastly, the last pellet is taken up in 3 ml of Liebovitz L-15/10% FBS and the cell suspension obtained is subjected to the cell count in step 3.

The cell count performed in a modified Neubauer chamber (Z359629 Sigma) allows calculating the final cell concentration and the cell yield per gram of liver. More in detail, the cell concentration in the final suspension (N° cells/ml) was estimated at around 1.4×10⁶ cells/ml and the cell yield (N°cells/g of liver) at around 2 ×10⁷ viable cells/g. The viability step follows in step 3, through cell count after staining the suspension with Trypan Blue. The viability of the isolated hepatocyte was calculated as follows and expressed in percentage: N° viable cells (not stained)/N° non-viable cells (blue stained). The viability estimation, calculated based on the % of hepatocytes excluded by Trypan blue, exceeded 95%.

Immediately after the count, the hepatocytes are resuspended in the Liebovitz L-15 medium supplemented with hormones, vitamins, amino acids and growth factors (Complete growth medium n° 3 in table n° 3). Then they are seeded and placed in culture directly in 96 well microplates (BD Falcon Primaria TC, 353872 BD Bioscenses), at a 3x10⁴ cells/cm² density (50µl/w), previously covered with a collagen solution (50µl/w of 0,01%; Collagen Type I, Sigma).

**Table 3**

| **Complete growth medium in Liebovitz L-15, n°3** | | |
|---|---|---|
| **Complete growth medium** | **Final concentration** | **Initila concentration** |
| L-Glu | 2mM | 200mM |
| NaPyr | 1mM | 100mM |
| D-Glucose | 5mM | 0.55 M (100g/L) |
| FBS | 10% | |
| Pen/Strep/Amph 1X | 100 µg/ml | 10000µg/ml (100x) |
| Gentamicin 1X | 0.05mg/ml (50 µg/ml) | 10mg/ml |
| Hepes | 10 mM | 1M |
| NaHCO₃ | 12 mM | 0.89 M |
| NaCl | 20 mM | 154 mM (0.9%) |
| EGF | 0.01 µg/ml (10 ng/ml) | 100 µg/ml |
| HGF | 0.005 µg/ml (5 ng/ml) | 5 µg/ml stock sol. |
| Amino acid mix | 0.1 mM (1%) | 100x |
| Vitamin mix | 0.1µM | 100x |
| ITS+3 | 500 ng/ml | 1mg/ml x insulin |

In order to identify the most efficient plating density to obtain a monolayer, a preliminary titration curve was performed: Various cell concentrations in complete culture medium (Complete growth medium, table n° 3) were seeded in 96 well microplates. The cell count was performed every 24 hours through the staining method (Crystal Violet assay). In such manner it was possible to calculate amount of cells to be plated in order to obtain a growth in a monolayer within the 72-96 ours subsequent to seeding.

The cells Seeded in microplates are cultured in a thermostated refrigerated CO₂ incubator in (Innova CO2-170 New Brunswick Scientific, PBI) at 18°C, in a gaseous atmosphere containing 3% of CO₂ and 97% of air (according to the growth studies carried out in our laboratory and taking into account the lower partial tension of carbon dioxide present in vivo in the teleostei with respect to the one required by the mammal cells) (step 3).

The first replacement of the medium is performed 12 hours after seeding, required for the adhesion of the cells to the type I collagen matrix, (step 3). 24 hours after the adhesion, two cold PBS 1X (200 µl/w) were performed followed by the addition of a complete medium (Complete growth medium, table n° 3) (step 3). Next follows step 4 of maintaining the hepatocyte in culture with a daily replacement of the complete growth medium. The maximum growth time calculated for hepatocytes Seededin T-25 flasks , in the presence of differentiation mediums, replacing the complete medium upon reaching the semiconfluence (60% confluence) (Differentiation medium, table n° 4) (step 4), was 2 months. The osmolarity of the culture medium was uniformed to the serum osmolality of *Sparus aurata* and of *Dicentrarchus labrax* equivalent to 353.33 mOsm with 0.35% NaCl (Faisal M. et al., 1995; Lopez-Castejon G. et al., 2008).

**Table 4**

| **Differentiation medium in Liebovitz L-15, n° 4** | | |
|---|---|---|
| **Complete growth medium** | **Final concentration** | **Initial concentration** |
| L-Glu | 2mM | 200mM |
| NaPyr | 1mM | 100mM |
| D-Glucose | 5mM | 0.55 M (100g/L) |
| FBS | 10% | |
| Pen/Strep/Amph 1X | 100 µg/ml | 10000µg/ml (100x) |
| Gentamicin 1X | 0.05mg/ml (50 µg/ml) | 10mg/ml |
| Hepes | 10 mM | 1M |
| NaHCO₃ | 12 mM | 0.89 M |
| NaCl | 20 mM | 154 mM (0.9%) |
| EGF | 0.01 µg/ml (10 ng/ml) | 100 µg/ml |
| HGF | 0.005 µg/ml (5 ng/ml) | 5 µg/ml stock sol. |
| Desametasone | 0.1 µg/ml | 20 µg/ml (200x) |
| Hydrocortisone | 2 µg/ml | 2 mg/ml |
| Ascorbic acid | 0.1 mM | 56 mM |
| DMSO | 0.04% | 2% |
| TTS+3 | 500 ng/ml | 1mg/ml x insulin |

Such process has various advantages, in that it allows to perform studies *in vitro* even on those marine fish species, the most common in the Mediterranean sea, less studied up to date, and to transfer the know how thus obtained from in vitro to in vivo.

Further advantage lies in the simple and rapid access for industrial application. As a matter of fact, the most renowned stabilised cell lines and/or primary cell culture supplying international companies currently do not have any type of cells of the abovementioned water species. On the contrary, hepatocytes solely derived from mammal and some freshwater vertebrates species are easily available.

In particular, the European sea bass and Gilthead Sea bream hepatocytes are immediately available for distribution in the market in different plate culture formats, for example, the most versatile format is represented by the 96 well microplate, in which they reach the monolayer growth within the 72 hours subsequent to seeding and isolation.

The fields of application of such process *in vitro* are vast and considerable:
- Zoocoltures: culture modelling *in vitro* with the aim of studying growth and metabolic parameters of the marine fish species; screening of stress biomarkers in intensive rearing conditions; evaluating effects caused at cell level by contaminants and/or substances present in the feeding products, such as antioxidants, vegetable oils, etc.;
- Biotechnological applications: proteomics and functional genomics studies;
- Toxycology: preparing ecotoxitocological, citotoxycological e genotoxicological assays to investigate on the effects caused by chemical pollutants in the sublethal doses; studies on cytotoxicity and genotoxicity caused by mycotoxins such as potential contamminants present in the feeding products of the reared marine fish species.
- Pharmacology: Studies of exposure to different therapeutic molecules;
- Genetics: mutant analysis and complementation tests, mapping and transfectioned with exogenous DNA;
- Oncology: characterisation of tumours, identification of carcinogen substances, experimentation of anti-tumour molecules.
- Public health: application of *in vitro* models as an alternative to animal *in vivo* biological test;
- Immunology: production of recombinant proteins, immunostimulants and monoclonal antibodies;
- Infectious diseases: diagnostic studies (isolation and viral typing), preparing vaccines for controlling viral pathologies in aquaculture.

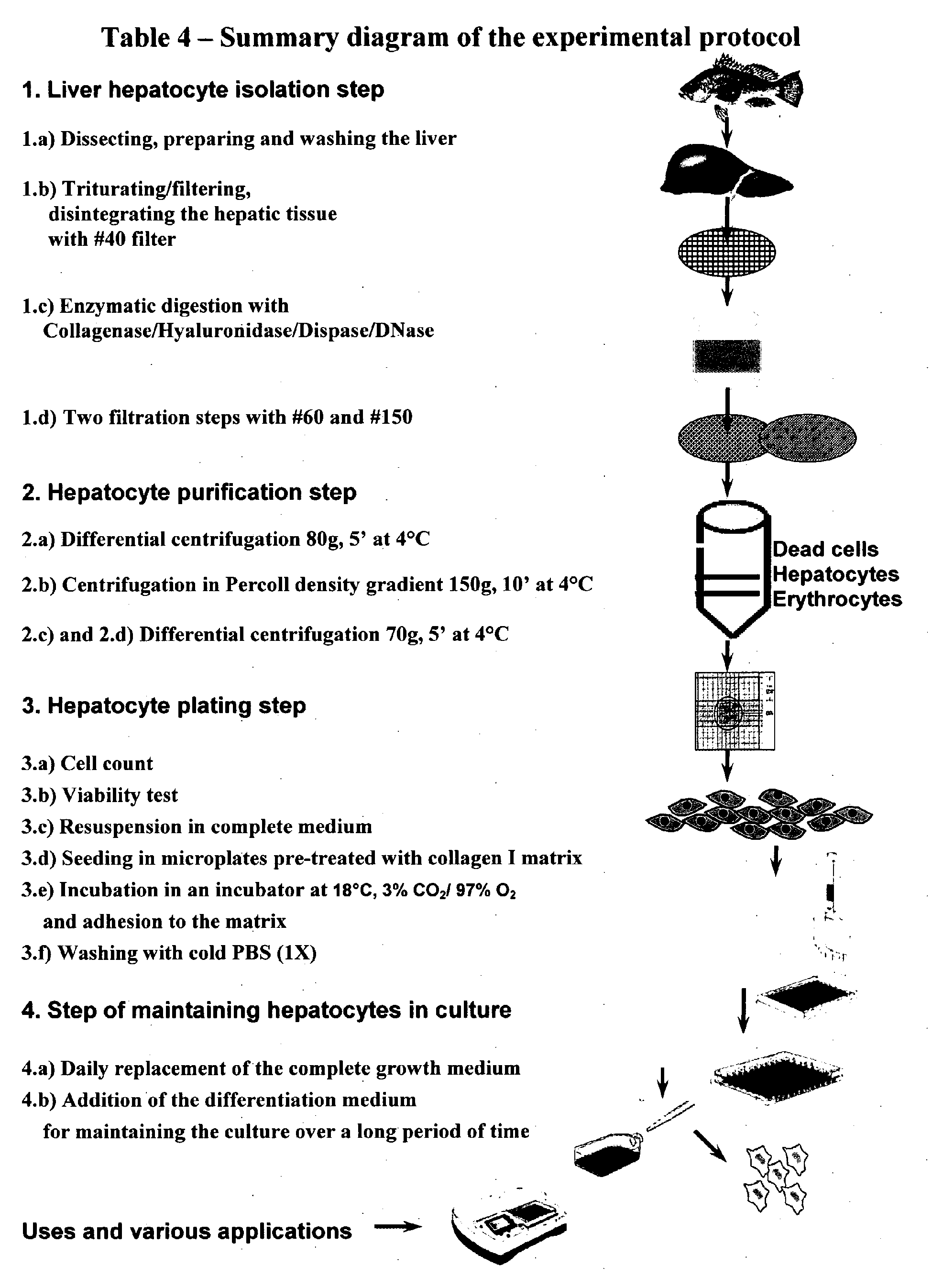

## Claims

1. Biotechnological process for preparing primary hepatocyte cultures from the liver of sea species, in particular *D. labrax* and *S. aurata*, **characterised by** a step (1) of isolating hepatoctyes from the liver followed by a step (2) of purifying hepatocytes obtained from step (1) with separation from non-parenchyma cells through at least one centrifugation stage then a step (3) of plating hepatocytes and lastly a step (4) of maintaining the hepatocytes in culture.

2. Process according to claim 1, wherein said step of isolating the hepatocyte from the liver (1) comprises dissecting, preparing and washing the liver (1.a) followed by triturating and filtering the explanted hepatic tissue using a steel filter (1.b) and the enzymatic digestion (1.c) and lastly at least one filtration on steel filters (1.d).

3. Process according to claim 1 or 2, wherein said hepatocyte purification step (2) comprises purification through at least one differential centrifugation (2.a) followed by at least one centrifugation in Percoll density gradient (2.b) and lastly at least one further differential centrifugation (2.c), (2.d).

4. Process according to claim 1, 2 or 3, wherein said hepatocyte plating step (3) comprises cell count (3.a), followed by a viability test (3.b), the resuspension of the hepatic cells in complete medium (3.c), seeding in microplates pre-treated with collagen matrix (3.d), then incubation in a refrigerated CO2 incubator and adhesion to the collagen matrix (3.e), and lastly the at least one washing in cold phosphate buffered saline (PBS) (3.f).

5. Process according to claims 1, 2, 3 or 4, wherein said step of maintaining hepatic cells in culture (4) comprises daily replacement of the complete growth medium (4.a) repeated for at least one day until reaching semiconfluence and subsequent addition of the differentiation medium for maintaining the differentiated phenotype in culture over a long period of time (4.b).

6. Process according to one of the preceding claims, wherein said dissecting, preparing and washing the liver activity (1.a) is **characterised by** at least one washing of the external surface of the liver by submerging it in a saline buffer free of calcium and magnesium ions, while the washing of the internal hepatic tissue is performed by means of a syringe until complete clarification of blood.

7. Process according to one of the preceding claims, wherein said activity of triturating and filtering the explanted hepatic tissue using a #40 steel filter (2.b) is **characterised in that** it allows tissue disintegration both by means of at least one stainless steel filter mounted on a sterile #40 steel colander, and by means of at least one glass pestle adapted to press the hepatic masses with rotational movements and furthermore, by adding small regular doses of the digestion buffer.

8. Process according to one of the preceding claims, wherein said enzymatic digestion activity (1.c) is **characterised in that** it is performed by means of a mix of enzymes (Collagenase, Hyaluronidase, Dispase, DNase) with different enzymatic activity and final concentration, with incubation under a low stirring on an orbital shaker and finally with the addition of Liebovitz L-15/FBS 10% to block the residual enzymatic activity.

9. Process according to one of the preceding claims, wherein said filtration on steel filters (1.d) is **characterised, in** a preferred implementation, by two passages on a #60 steel filter first followed by a #150 filter to allow fine disassociation of the cell aggregates.

10. Process according to one of the preceding claims, wherein said differential centrifugation activity (2.a), in a preferred implementation, is **characterised by** at least one washing of the disassociated cells by means of centrifugation at 80g, 5' at 4°C, in order to remove the connective microfilaments, the broken cells fragments and the erythrocytes.

11. Process according to one of the preceding claims, wherein said centrifugation activity in Percoll density gradient (2.b) **characterised in that** in a special implementation it has 150g, 10' at 4°C values in order to provide a clear stratification of cell bands in:
• A layer containing dead cells and fragments;
• A layer containing a white ring of isolated cells (hepatocytes);
• A layer containing a red ring of lysed erythrocytes positioned in close contact with the Percoll.

12. Process according to one of the preceding claims, wherein said differential centrifugation activity (2.c), (2.d) is **characterised in that** in a special implementation its has 70g, 5' at 4°C values, and it is performed at least once with the aim of clarifying and cleaning the final pellet.

13. Process according to one of the preceding claims, wherein said viability control (3.b) is performed by means of cell count after staining the suspension wut Trypan blue, performed based on the percentage of hepatocytes excluded by Trypan blue.

14. Process according to one of the preceding claims, wherein said activity of resuspension of hepatic cells in complete medium (3.c) is **characterised in that** it is supplemented with hormones, vitamins, amino acids and growth factors.

15. Process according to one of the preceding claims, wherein said incubation activity in a CO2 incubator (3.e) is **characterised in that**, in a special implementation, it is refrigerated at 18°C, in 3% CO2/97%O2 to allow the isolated hepatocytes to adhere to the collagen matrix within the first 12 hours from seeding in the microplate (3.d).

16. Process according to one of the preceding claims, wherein said washing operation activities using cold phosphate buffer solution (PBS) (3.f) is **characterised in that**, in a special implementation, after 24 hours from the adhesion (3.e), its is followed by addition of a complete growth medium and incubation in a CO2 refrigerated incubator at 18°C, in 3% CO2/97%O2.

17. Use of the biotechnological process for preparing primary hepatocyte cultures from the liver of marine water species, according to any one of the preceding claims, for application in zooculture, biotechnology, toxicology, pharmacology, genetics, oncology, public health, immunology, diagnosis of infectious diseases.
